(19) Europäisches Patentamt
European Patent Office
Office européen des brevets

(11) Publication number: 0 022 653 A1

# (12) EUROPEAN PATENT APPLICATION

(21) Application number: 80302315.9

(22) Date of filing: 09.07.80

(51) Int. Cl.³: **C 07 C 127/00**, C 07 D 273/04, C 07 D 295/20, A 01 N 47/28, A 01 N 43/36, A 01 N 43/88

(30) Priority: 10.07.79 US 56179

(43) Date of publication of application: 21.01.81 Bulletin 81/3

(84) Designated Contracting States: CH DE FR GB IT LI NL

(71) Applicant: Rohm and Haas Company, Independence Mall West, Philadelphia, Pennsylvania 19105 (US)

(72) Inventor: Smolanoff, Joel Robert, 9 Hickory Lane, Chalfont Pennsylvania, 18914 (US)

(74) Representative: Wood, Peter Worsley Spencer et al, Rohm and Haas Company Patent Department Chesterfield House Barter Street, London, WC1A 2TP (GB)

## (54) Novel urea derivatives, arthropod repellent compositions containing them and their use for repelling arthropods.

(57) Novel urea derivatives are provided which are of the formula

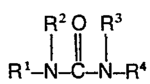

wherein $R^1$ is alkenyl or alkynyl; $R^2$ is alkenyl, alkynyl, cycloalkyl or phenyl($C_1$–$C_8$)alkyl; $R^3$ is hydrogen; $R^4$ is alkyl, alkoxycarbonylalkyl or cycloalkyl; or $R^2$ and $R^3$ are joined to form a dimethyleneoxy group; or $R^3$ and $R^4$ are joined and form, together with the attached nitrogen atom, a 5- to 7-membered heterocyclic ring.

The novel urea derivatives find use as arthropod repellents either when used alone or in conjunction with an inert carrier.

EP 0 022 653 A1

# NOVEL UREA DERIVATIVES, ARTHROPOD REPELLENT COMPOSITIONS CONTAINING THEM AND THEIR USE FOR REPELLING ARTHROPODS

This invention concerns novel urea derivatives, arthropod repellent compositions containing them and their use for repelling arthropods.

The search for arthropod repellents, particularly insect repellents, which have a combination of excellent repellency, high residual activity and essentially no toxicity is a continuing one due to recognition of the possible toxicity to animals or humans of many known arthropodicides. Since long lasting repellents provide essentially the same results as an arthropodicide and they also avoid the toxicity problems, compounds having these effects are in great demand.

The novel compounds of this invention have the following structural formula:

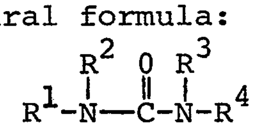

(I)

wherein $R^1$ is alkenyl (e.g. alkenyl containing at least 3 carbon atoms such as $(C_3-C_7)$alkenyl, examples being allyl, 2-methylallyl, 1-butenyl, 1-pentenyl, 1-hexenyl and 1-heptenyl); alkynyl (e.g. alkynyl of at least 3 carbon atoms such as $(C_3-C_7)$alkynyl, examples being 2-propynyl, 2-butynyl, 2-pentynyl, 2-hexynyl and 2-heptynyl); $R^2$ is $(C_1-C_8)$alkyl, alkenyl (examples being those given above for $R^1$), alkynyl (examples being those given above for $R^1$), cycloalkyl (e.g. $(C_5-C_7)$cycloalkyl such as cyclopentyl, cyclohexyl and cycloheptyl), phenyl$(C_1-C_8)$alkyl (particularly phenyl$(C_1-C_3$ or $C_4)$alkyl such as benzyl, phenethyl and phenylpropyl); $R^3$ (when not joined to $R^2$ or $R^4$) is hydrogen; $R^4$ is alkyl

(e.g. $(C_1-C_8)$alkyl of from 1 to 8 carbon atoms such as methyl, ethyl, propyl, butyl, pentyl, hexyl, heptyl and octyl, including all the possible isomers thereof), cycloalkyl (e.g. cyclo$(C_5-C_7)$alkyl (examples being those given above for $R^2$), alkoxycarbonylalkyl (e.g. alkoxycarbonylalkyl having from 1 to 8, e.g. 1 to 3 or 4, carbon atoms in each of the alkoxy and alkyl moities such as methoxycarbonylmethyl, ethoxy-carbonylmethyl, propoxycarbonylmethyl, butoxycarbonylmethyl; or $R^2$ and $R^3$ are joined in which case they represent dimethyleneoxy (i.e. $-CH_2OCH_2-$); or $R^3$ and $R^4$ are joined in which case they form, together with the attached nitrogen atom, a 5- to 7-membered heterocyclic ring such as a pyrrolidinyl or piperidyl residue.

The novel compounds of the invention provide a useful choice, in comparison with known repellents, in that they have one or more of the following properties, viz repellency activity for an acceptable period of time, ease and/or cheapness of manufacture and acceptably low mammalian toxicity.

A preferred class of compounds are those of the formula:

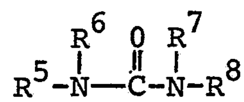

(Ia)

wherein $R^5$ is allyl, 2-methylallyl or 2-propynyl; $R^6$ is allyl, 2-methylallyl or cyclohexyl; $R^7$ is hydrogen; $R^8$ is $(C_1-C_8)$alkyl or alkoxycarbonylmethyl having 1 to 8, i.e. 1 to 3 or 4, carbon atoms in the alkoxy moiety; or $R^6$ and $R^7$ are joined to form dimethyleneoxy or $R^7$ and $R^8$ are joined and form, together with the attached nitrogen atom, a pyrrolidinyl residue. Especially preferred are those compounds wherein one or both of $R^5$ and $R^6$ is or are

allyl or 2-methylallyl as such compounds exhibit particularly good repellent action. Compounds having particularly good repellent action which may be singled out for mention are compounds of Formula Ia wherein (1) $R^5$ and $R^6$ are the same and are allyl or 2-methallyl, $R^7$ is hydrogen and $R^8$ is n-propyl or n-butyl, (2) $R^5$ is allyl, $R^6$ is cyclohexyl, $R^7$ is hydrogen and $R^8$ is $(C_1-C_3)$alkyl or ethoxycarbonylmethyl, (3) $R^5$ is allyl, $R^6$ and $R^7$ are joined to form a dimethyleneoxy group and $R^8$ is $(C_4-C_6)$alkyl and (4) $R^5$ is 2-methallyl, $R^6$ and $R^7$ are joined to form a dimethyleneoxy group and $R^8$ is $(C_3-C_8)$alkyl.

Compounds of Formula I (excluding those compounds where $R^2$ and $R^3$ are joined to form dimethyleneoxy and those where $R^3$ and $R^4$ are joined to form a heterocycle) can be prepared by treating an appropriately substituted amine (II, infra) with an appropriately substituted isocyanate (III, infra). The following reaction scheme shows this process:

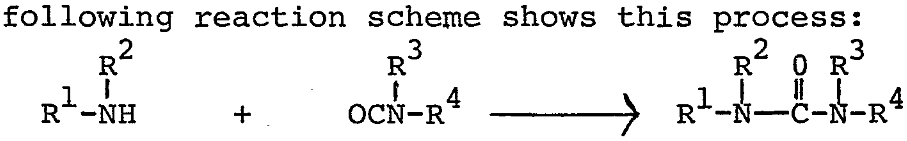

(II) (III) (I)

wherein $R^1$, $R^2$, $R^3$ and $R^4$ are as defined above except that $R^2$ is not joined to $R^3$ and $R^3$ is not joined to $R^4$.

The reaction with the amine (II, supra) and the isocyanate (III, supra) may be conducted in any solvent which is inert or substantially inert to the reactants such as ethers including diethylether or tetrahydrofuran. The reaction may be conducted at a temperature in the range of from 0 to 100°C for a period of time from about 15 minutes to about 3 hours; however, the reaction is generally initiated at room temperature and conducted at room temperature for a period of time of about 1 hour.

Those compounds wherein $R^3$ and $R^4$ are joined

together with the nitrogen atom to which they are attached to form a heterocyclic ring are prepared by treating an appropriately substituted amine with an appropriately substituted acid chloride. The following reaction scheme shows this process:

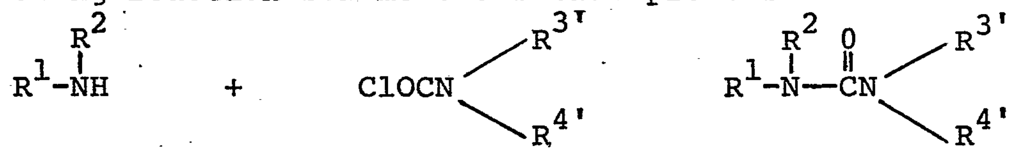

(II) (IV) (Ia)

where $R^1$ and $R^2$ are as defined above and $R^{3'}$ and $R^{4'}$ are joined and form, together with the attached nitrogen, a 5 to 7-membered heterocyclic ring.

The reaction of the amine (II, supra) with the acid chloride (IV, supra) may be conducted in any solvent which is inert or substantially inert to the reactant such as toluene, benzene, hexane, acentonitrile or carbon tetrachloride. The reaction may be conducted at a temperature in the range of from 0 to 100°C for a period of time of from 1 to 5 hours; however, the reaction is generally initiated at room temperature and conducted at room temperature for about 3 hours.

Those compounds wherein $R^2$ and $R^3$ are joined to form a radical of the formula, $-CH_2OCH_2-$ are prepared by reacting the compounds of Formula I wherein $R^2$ and $R^3$ are hydrogen with paraformaldehyde in the presence of an organic acid such as p-toluene sulfonic acid and azeotroping the water formed during the reaction by employing an appropriate solvent such as benzene or chloroform. This reaction is shown below:

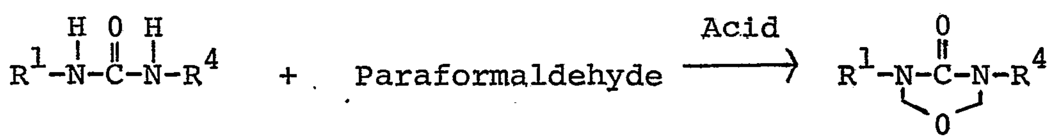


(Ib)

The compounds of Formula I can also be prepared by other methods such as are disclosed in the literature for the preparation of compounds having an analogous structure.

The compounds of this invention can be employed over a wide range of concentration in a variety of carriers or diluents conventionally used in the art.

The amount of compound employed in the insect repellent compositions can vary between from about 0.1 to about 90 weight percent basis of the weight of the composition and will depend upon the intended use. Usually, the compositions contain between about 0.1 to about 10 weight percent of one or more of the compounds of Formula I and the compound is usually in intimate mixture with the carrier.

When it is desired to use the insect repellent composition directly (i.e., without further dilution), the amount of the compound used can usually vary from between about 0.1 to 5.0 weight percent of the composition. When it is desired to formulate a concentrated composition, i.e., one suitable for dilution prior to end-use, the compounds will usually be present in the composition in an amount of from about 0.5 to about 90 weight percent.

The carrier employed can be any carrier conventionally used in insect repellent formulations. The carrier should also be one that will not be harmful to the environment. The carrier can be any one of a variety of organic and inorganic liquid, solid, or semi-solid carriers or carrier formulations conventionally used in insect repellent products and can be a mixture of such carriers.

Examples of organic liquid carriers include liquid aliphatic hydrocarbons such as pentane, hexane, heptane, nonane, decane and their analogues, as well as liquid

aromatic hydrocarbons. Examples of other liquid hydrocarbons include oils produced by the distillation of coal and the distillation of various types and grades of petrochemical stocks including kerosene oils which are obtained by fractional distillation of petroleum at between 84°C and 130°C and which usually have a flash point between 18°C and 32°C.

Other petroleum oils include those generally referred to in the art as agricultural spray oils which are light and medium spray oils consisting of the middle fractions in the distillation of petroleum and have a viscosity in the range of from about 40 to 85 sec. Saybolt at 4°C and are only slightly volatile. These oils are usually highly refined and contain only minute amounts of unsaturated compounds as measured by standard sulfonation tests. The customary sulfonation range of such oils is between 90% and 94% of unsulfonatable residue. These oils are paraffin oils and can be emulsified with water and an emulsifier and diluted to lower concentrations and used as sprays. Tall oils obtained from sulfate digestion of wood pulp, like paraffin oils, also can be employed.

In addition to the above-mentioned liquid hydrocarbons, the carrier can contain conventional emulsifying agents (e.g., a non-ionic surfactant such as an ethylene oxide condensate of octyl phenol or an anionic surfactant such as an alkali metal salt of an alkylbenzenesulfonic acid). Such emulsifiers are used to permit the composition to be dispersed in and diluted with water for end-use application.

When paraffin oils are employed as carriers in the insect repellent compositions of this invention, they are usually used in conjunction with an emulsifier, the mixture being diluted with water immediately prior to

the end-use application. Other suitable paraffin oils, particularly those used with emulsions, are referred to in the art as heavy paraffin oils and usually have a viscosity greater than 85 sec. Saybolt at 4°C.

Other advantageous organic liquid carriers can include liquid terpene hydrocarbons and terpene alcohols such as alpha-pinene, dipentene and terpineol. Still other liquid carriers include organic solvents such as aliphatic and aromatic alcohols, esters, aldehydes, and ketones. Aliphatic monohydric alcohols include methyl, ethyl, n-propyl, isopropyl, n-butyl, sec-butyl and t-butyl alcohols. Suitable dihydric alcohols include glycols such as ethylene and propylene glycol and the pinacols (alcohols having the empirical formula: $C_6H_{12}(OH)_2$). Suitable polyhydroxy alcohols include glycerol, arabitol, erythritol and sorbitol. Suitable cyclic alcohols include cyclopentyl and cyclohexyl alcohols.

Conventional aromatic and aliphatic esters, aldehydes and ketones can be employed and are usually used in combination with the above-mentioned alcohols. Still other liquid carriers including high-boiling petroleum products, such as mineral oil and higher alcohols, such as cetyl alcohol can also be employed. Additionally, conventional "stabilizers" or "synergizers" such as t-butyl sulfinyl dimethyl dithiocarbamate, can be employed in conjunction with, or as a component of, the carriers comprising the compositions of this invention.

Solid carriers which can be used in the compositions of this invention include finely divided organic and inorganic solid materials. Suitable finely divided solid inorganic carriers include siliceous minerals such as clay, including bentonite,

attapulgite, fuller's earth, diatomaceous earth, kaolin, mica, talc or finely divided quartz, as well as synthetically prepared siliceous materials, such as silica aerogels and precipitated and fume silicas.

Examples of finely divided solid organic materials include cellulose, sawdust and synthetic organic polymers.

Examples of semi-solid carriers include petroleum jelly and lanolin and mixtures of liquid and solid carriers which provide semi-solid carrier products.

The repellent compositions can be employed as such or can be diluted with suitable liquids or solids to repel common flying and crawling insect pests, such as roaches, moths, house and stable flies, termites, flour beetles, bean beetles, weevils, ticks, chinch bugs, lice, ants, chiggers and mosquitoes. The compositions, when used to contact an insect environment, effectively repel the insects. By way of example, one advantageous embodiment of a composition of this invention comprises from about 0.1 to about 90 percent, preferably 0.1 to about 10 percent by weight of an active compound falling within the scope of this invention, in intimate mixture with one or more of the above-mentioned carriers.

Insect pests can be repelled by contacting the surfaces on which the insects may alight or crawl such as clothing, tents or skin and with a liquid, solid or semi-solid composition. The contact can be accomplished directly (e.g., by atomizing the composition into the air as a liquid or as a dust so that the material will fall on the desired surface).

By way of further example, insect-infested animals, such as dogs with fleas or poultry with lice, cows with ticks may be treated with the insect repellent compositions by contacting the fur and/or feathers and the lice, fleas and ticks contained therein, thereby

ending the insect infestation. Livestock such as cattle, sheep, horses, ponies and goats may be treated by spraying the animals with a liquid composition such as one containing the active ingredient water, a co-solvent if necessary, and a surfactant or by a dipping procedure using such a liquid composition. Also, granaries and silos can be treated with the compositions of this invention, prior to grain storage, to prevent beetle, weevil, and other insect infestations in the grain to be subsequently stored. Food packaging elements or containers, including fibre, cardboard, wooden shipping containers, storage bins and flour sacks, can be treated with the compositions of this invention to prevent insect infestation.

The following examples illustrate the preparation of the compounds of this invention.

Example 1

N,N-Diallyl-N'-ethylurea

To a 100 ml 3-necked flask fitted with a stirrer, condenser and dropping funnel is added diallylamine (9.7 g; 0.1 moles) and diethylether (50 ml). Ethyl isocyanate (7.1 g; 0.1 moles) in diethyl ether (10 ml) is then added dropwise with cooling. The reaction mixture is stirred at room temperature for one hour and the ether removed to afford 16 g of N,N-diallyl-N'-ethylurea as a yellow oil.

By following substantially the procedure of Example 1 and by employing the starting materials as shown in Table I, the following compounds are prepared according to the reaction scheme given above Table I.

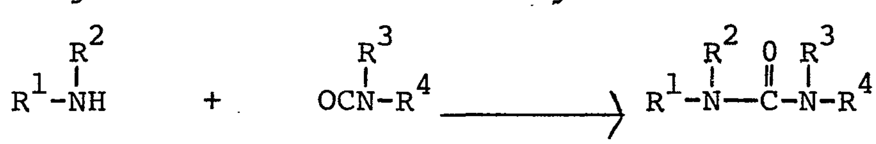

TABLE I

| Ex. No. | $R^1$ | $R^2$ | $R^3$ | $R^4$ |
|---|---|---|---|---|
| 2 | $CH_2=CHCH_2-$ | $CH_2=CHCH_2-$ | H | $-C_3H_7-\underline{n}$ |
| 3 | " | " | H | $-C_4H_9-\underline{n}$ |
| 4 | " | " | H | $-CH_2CO_2C_2H_5$ |
| 5 | " | " | H | $-CH_2CO_2C_4H_9-\underline{n}$ |
| 6 | $CH_3$<br>\|<br>$CH_2=CCH_2-$ | $CH_3$<br>\|<br>$CH_2=CCH_2-$ | H | $-C_3H_7-\underline{n}$ |
| 7 | " | " | H | $-C_4H_9-\underline{n}$ |
| 8 | " | " | H | $-CH_2CO_2C_2H_5$ |
| 9 | " | " | H | $-CH_2CO_2C_4H_9-\underline{n}$ |
| 10 | $CH\equiv CCH_2-$ | $CH_3$ | H | $-C_3H_7-\underline{n}$ |
| 11 | " | " | H | $-C_4H_9-\underline{n}$ |
| 12 | " | " | H | $-CH_2CO_2C_2H_5$ |
| 13 | " | " | H | $-CH_2CO_2C_4H_9-\underline{n}$ |
| 14 | $CH_2=CHCH_2-$ | 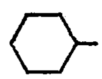 | H | $CH_3$ |
| 15 | " | " | H | $-C_2H_5$ |
| 16 | " | " | H | $-C_3H_7-\underline{n}$ |
| 17 | " | " | H | $-CH_2CO_2C_2H_5$ |
| 18 | " | " | H | $-CH_2CO_2C_4H_9-\underline{n}$ |

Example 19

N-Allyl-N-cyclohexyl-N'-tetramethyleneurea

In a 100 ml 3-necked round bottom flask equipped with a stirrer, condenser and a dropping funnel is added carbamoyl chloride (9.3 g; 0.07 moles), triethylamine (7.1 g; 0.0 moles) and

toluene (15 ml). To this solution is added dropwise allylcyclohexylamine (10.0 g; 0.07 moles). Reaction mixture is warmed to 45°C and then stirred at room temperature for 3 hours. The amine hydrochloride is filtered off and the solvent removed to afford 13 g of N-(allyl)-N-cyclohexenyl-N'-tetramethyleneurea.

<u>Example 20</u>

<u>N,N-bis-(2-methallyl)-N'-tetramethyleneurea</u>

In a 100 ml 3-necked round bottom flask fitted with a stirrer, condenser and dropping funnel is added carbamoyl chloride (5.3 g; 0.04 moles) triethylamine (4.0 g; 0.04 moles) and toluene (60 ml). To this solution is added dropwise bis-(2-methallylamine) (5.0 g; 0.04 moles). The reaction mixture is warmed to 45°C and stirred at room temperature for 3 hours. The amine hydrochloride is collected by filtration and the toluene removed to afford 8 g of N,N-bis-(2-methallyl)-N'-tetramethyleneurea.

By following substantially the procedure described in Examples 19 and 20 and by employing the starting materials shown in Table II, the following compounds are prepared according to the reaction scheme given above Table II.

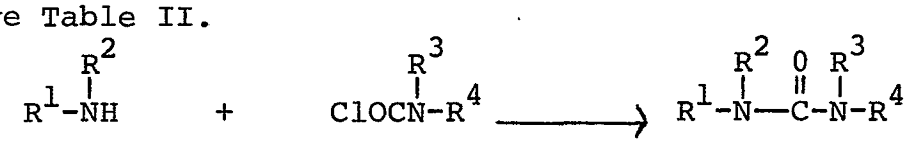

<u>TABLE II</u>

| Ex. No. | $R^1$ | $R^2$ | $R^3 + R^4$ |
|---|---|---|---|
| 21 | 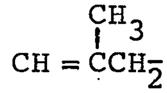 | $CH_3$ | $-CH_2CH_2CH_2CH_2-$ |
| 22 | " | $C_6H_5CH_2$ | " |

Example 23

Tetrahydro-3-n-hexyl-5-allyl-4H-1,3,5-oxadiazin-4-one

In a 300 ml 3-necked flask equipped with a stirrer, condenser and dropping funnel is added N-n-hexyl-N-allylurea (18.4 g; 0.1 moles), paraformaldehyde (6.0 g; 0.2 moles), p-toluenesulfonic acid (1.0 g) and chloroform (150 ml). The reaction mixture is heated to reflux and 1.8 ml of water is collected. The reaction mixture is cooled to room temperature, neutralized with sodium hydroxide and washed with water. The chloroform solution is dried over magnesium sulfate, and filtered. The chloroform is removed and the residue distilled to afford 15 g of tetrahydro-3-n-hexyl-5-allyl-4H-1,3,5-oxadiazin-4-one b.p. 105-112°C/0.6 mm.

By following substantially the procedure of Example 22 and by employing the starting materials shown in Table III, the following compounds are prepared according to the reaction scheme given above Table III.

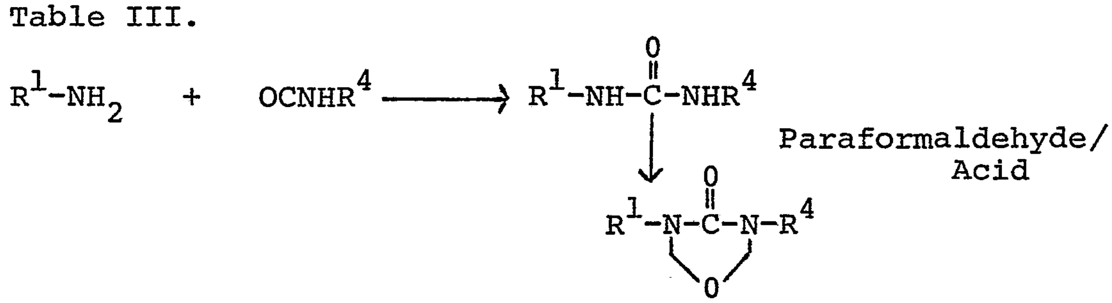


TABLE III

| Ex. No. | $R^1$ | $R^4$ | Boiling Point °C/mm |
|---|---|---|---|
| 24 | $CH_2=CHCH_2-$ | $-C_2H_5$ | 95/0.5 |
| 25 | " | $-C_3H_7$-n | 80/1 |
| 26 | " | $-C_4H_9$-n | 90/.75 |
| 27 | " | $-(CH_2)_4CH_3$ | - |

TABLE III (continued)

| Ex. No. | $R^1$ | $R^4$ | Boiling Point °C/mm |
|---|---|---|---|
| 28 | " | 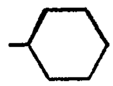 | 113/.5 |
| 29 | 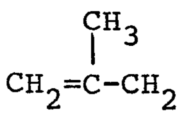 | $-C_2H_5$ | 75/1.5 |
| 30 | " | $-C_3H_7$-n | 73/1.7 |
| 31 | " | $-C_4H_9$-n | 61/1.7 |
| 32 | " | $-(CH_2)_7CH_3$ | 93/.5 |
| 33. | " | 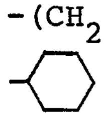 | 75/1.5 |

The following test description and results illustrate the use of the novel compounds of this invention.

Repellency Screen

Male albino guinea pigs (Perfection Breeders) are divided into groups of 2 each and placed into individual cages in a rodent battery equipped with an automatic watering system. Individual animal body weights ranged from 450 to 600 g. Feed and water are provided ad libitum. Guinea pigs are prepared for testing by clipping a patch of hair from the back with a size 10 clipper blade. This permits a residual amount of hair to be left on the animal.

Test compounds are formulated as 5% solutions in acetone. A 2.5 ml volume of test solution is applied with a medicine dropper pipette to an area on the animal's back measuring approximately 7 cm x 5 cm. This application results in a deposit rate of 3.5 mg/$cm^2$. Two guinea pigs are treated with each compound. The test animal is anaesthetized with sodium pentobarbital administered intraperitoneally

at the rate of 35 mg/kg and is placed in a cylindrical plastic cage with only the treated portion of the back exposed. The masked animal is introduced into an insect cage filled with either starved stable flies or yellow fever mosquitoes. Approximately 500-1000 insects were used as the challenge. The treated guinea pig is exposed to the test insects for a 5-10 minute period initially and at 3 hours post-treatment and then on a daily basis until the repellency activity of the compound terminates. The residual repellency activity of a compound is regarded as terminated when three or more test insects fed on the guinea pig during the exposure period. N.A. means not active at the test dose.

| Compound of Example No. | Protection Time | |
|---|---|---|
| | Stable Fly | Yellow Fever Mosquito |
| 1 | 3 Hours (H) | 3 Hours (H) |
| 2 | 1 Day (D) | 1 Day (D) |
| 3 | 2D | 3D |
| 4 | 0.5H | NA |
| 5 | 1D | 1D |
| 6 | 2D | 2D |
| 7 | $4^+$D | $4^+$D |
| 8 | 0.5H | 0.5H |
| 9 | 0.5H | 0.5H |
| 10 | 0.5H | 0.5H |
| 11 | 3H | 1D |
| 12 | 3H | 3H |
| 13 | 0.5H | 0.5H |
| 14 | $4^+$D | $4^+$D |
| 15 | $4^+$D | $4^+$D |
| 16 | $4^+$D | $4^+$D |
| 17 | $4^+$D | $4^+$D |
| 18 | 0.5H | 0.5H |

| Compound of Example No. | Protection Time Stable Fly | Yellow Fever Mosquito |
|---|---|---|
| 23 | 4D | 5D |
| 24 | 3H | 3H |
| 25 | 3H | 3H |
| 26 | 2D | 2D |
| 27 | 3D | $4^{+}$D |
| 28 | 1D | 1D |
| 29 | 1D | 1D |
| 30 | 1D | 1D |
| 31 | 2D | 3D |
| 32 | 3D | $4^{+}$D |
| 33 | 1D | 1D |

In conclusion, reference is made to a sub-class of compounds of Formula I wherein, when $R^1$ and $R^2$ are both allyl and $R^3$ is hydrogen, $R^4$ is butyl, particularly _n_-butyl, alkoxycarbonylalkyl or cycloalkyl.

Claims:

1. A compound of the formula

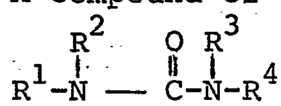

wherein $R^1$ is alkenyl or alkynyl;
$R^2$ is $(C_1-C_8)$alkyl, alkenyl, alkynyl, cycloalkyl or phenyl$(C_1-C_8)$alkyl;
$R^3$ is hydrogen;
$R^4$ is alkyl, alkoxycarbonylalkyl or cycloalkyl; or
$R^2$ and $R^3$ are joined to form a dimethyleneoxy group; or $R^3$ and $R^4$ are joined and form, together with the attached nitrogen atom, a 5- to 7-membered heterocyclic ring.

2. A compound as claimed in Claim 1 of the formula

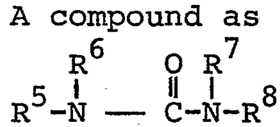

wherein $R^5$ is allyl, 2-methylallyl or 2-propynyl;
$R^6$ is allyl, 2-methylallyl or cyclohexyl;
$R^7$ is hydrogen;
$R^8$ is $(C_1-C_8)$alkyl, alkoxycarbonylmethyl having up to 8 carbon atoms in the alkoxy moiety or $(C_5-C_7)$cycloalkyl; or
$R^6$ and $R^7$ are joined to form a dimethyleneoxy group; or $R^7$ and $R^8$ are joined and form, together with the attached nitrogen atom, a pyrrolidinyl residue.

3. A compound as claimed in Claim 2, wherein either or both of $R^5$ and $R^6$ are allyl or 2-methylallyl.

4. A compound as claimed in Claim 1 which is N,N-bis-2-methylallyl-N'-*n*-propylurea.

5. A compound as claimed in Claim 1 which is N-allyl-N-cyclohexyl-N'-methylurea.

6. A compound as claimed in Claim 1 which is N-allyl-N-cyclohexyl-N'-propylurea.

7. A compound as claimed in Claim 1 which is N-allyl-N-cyclohexyl-N'-ethoxycarbonylmethylurea.

8. A compound as claimed in Claim 1 which is tetrahydro-3-hexyl-5-allyl-4H-1,3,5-oxadiazin-4-one.

9. A compound as claimed in Claim 1 which is tetrahydro-3-octyl-5-(2-methallyl)-4H-1,3,5-oxadiazin-4-one.

10. A method for repelling arthropods which comprises applying to an appropriate surface an effective repellent amount of a compound as claimed in any preceding claim.

11. A method as claimed in Claim 10 wherein the surface is the skin of livestock.

12. An insect repellent composition which contains a compound as claimed in any one of Claims 1 to 9 and an inert carrier.

European Patent Office

# EUROPEAN SEARCH REPORT

0022653

Application number

EP 80 30 2315

## DOCUMENTS CONSIDERED TO BE RELEVANT

| Category | Citation of document with indication, where appropriate, of relevant passages | Relevant to claim |
|---|---|---|
| A | FR - A - 1 269 348 (BASF)<br>* claims *<br>-------- | 1 |

**CLASSIFICATION OF THE APPLICATION (Int. Cl. 3)**

C 07 C 127/00
C 07 D 273/04
295/20
A 01 N 47/28
43/36
43/88

**TECHNICAL FIELDS SEARCHED (Int.Cl. 3)**

C 07 C 127/00
C 07 D 273/04
295/20

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | October 20, 1980 | GAUTIER |

EPO Form 1503.1 06.78